# EUROPEAN PATENT APPLICATION

(11) **EP 2 503 033 A1**
(43) Date of publication of application: **26.09.2012**
(21) Application number: 10831185.3
(22) Date of filing: 18.11.2010
(51) Int. Cl.: D01F 9/00, D01D 5/10, C12N 5/02, A61L 27/34, A61L 27/50

(54) **DEVELOPMENT OF BIOACTIVE ELECTROSPUN COATINGS FOR BIOMEDICAL APPLICATIONS**

(30) Priority: 20.11.2009 ES 200931034
(71) Applicant: Consejo Superior De Investigaciones Científicas (CSIC), 28006 Madrid (ES)
(72) Inventor: LAGARÓN CABELLO, José M., E-46980 Paterna (Valencia) (ES); TORRES GINER, Sergio, E-46980 Paterna (Valencia) (ES); GIMENO ALCAÑIZ, José V., E-46980 Paterna (Valencia) (ES); OCIO ZAPATA, María J., E-46980 Paterna (Valencia) (ES)
(74) Representative: Pons Ariño, Angel
(86) International application number: PCT/ES2010/070746
(87) International publication number: WO 2011/061378

(57) **Abstract**

The present invention relates to a bioactive composition obtained through the technique of electrospinning and comprising at least one polymer. Moreover, the invention also describes a procedure for the incorporation of such electrospun bioactive composition as a coating over a plastic matrix to obtain composite materials for use in both biodegradable and non-biodegradable biomedical implants and tissue engineering.

## Description

The present invention relates to a bioactive composition obtained through the technique of electrospinning and composed of at least one polymer. Furthermore, the invention also discloses a process for the incorporation of this composition as a coating over a plastic matrix to obtain composite materials for their use in biomedical implants and in tissue engineering.

### PRIOR ART

In recent years, considerable interest has arisen in the processing of polymeric materials on a nano- and submicrometric scale, and very in particular in the form of fibers. From among the different techniques that can be used for manufacturing these ultrafine fibers, the technique of electrospinning is currently the most widely used. This process, patented more than one hundred years ago, (US Patent, 705 691, 1902) is based on the creation of a high potential difference between a capillary, which contains a polymeric solution, and a metal support, whereon the fibers are deposited. Depending on the parameters of the equipment and characteristics of the polymeric solution, not only can fibers be obtained, but also other morphologies such as sheets, tubes and spheres (Torres-Giner S, Gimenez E, Lagaron JM. Characterization of the morphology and thermal properties of zein prolamine nanostructures obtained through electrospinning. Food Hydrocolloids 22 (4): 601, 2008). The main research activity of the use of this type of ultrafine structures is focussed on their use as bioactive materials, such as antimicrobial nanofibers (Torres-Giner S, Ocio MJ, Lagaron JM. Development of active antimicrobial fiber-based chitosan polysaccharide nanostructures using electrospinning. Engineering of Life Science 8 (3): 303, 2008; Torres-Giner S, Ocio MJ, Lagaron JM. Novel antimicrobial ultrathin structures of zein / chitosan blends obtained through electrospinning. Carbohydrate Polymers 77 (2): 261, 2009), submicroencapsulation of functional food (Fernández A, Torres-Giner S, Lagaron JM. Novel route to stabilization of bioactive antioxidants by encapsulation in electrospun fibers of zein prolamine. Food Hydrocolloids 23 (5): 1427, 2009) and fundamentally, and more recently, as supports for cellular growth (Torres-Giner S, Gimeno-Alcañiz JV, Ocio MJ, Lagaron JM. Comparative performance of electrospun collagen nanofibers cross-linked by means of different methods. ACS Applied Materials & Interfaces 1 (1): 218, 2009).

Due to the reduced diameter of the electrospun fibers, largely submicrometric, the coatings based on networks of these fibers have a high morphological similarity with the surface of tissues such as bones, skin, muscles, as well as the walls of certain organs. Furthermore, these fibers may be biocompatible and biodegradable, so that if they are additionally not cytotoxic (not harmful for the cells), they can also be metabolically resorbable naturally by the body. In this way, the biological functioning of a tissue, which is regulated by certain biological signals, may be controlled to favour the cellular activity on the surface of these materials. *In vitro* studies have been able to demonstrate that the growth of cell lines on electrospun coatings is favoured (Torres-Giner S, Gimeno-Alcañiz JV, Ocio MJ, Lagaron JM. Comparative performance of electrospun collagen nanofibers cross-linked by means of different methods. ACS Applied Materials & Interfaces 1 (1): 218, 2009).

The final objective of electrospun coatings in the field of tissue engineering is generally that of producing a support or scaffold for cellular growth. In this way, damaged organs and tissues can be partially coated by electrospun fibers to temporarily replace them until the actual cells are capable of again populating and synthesizing the original tissue. With the objective of imitating the chemical composition of the biological cellular tissues and walls, coatings have been patented consisting of proteins, especially those from the extracellular matrix of mammals, such as collagen or elastin (US Patent 0213389 A1, 2008). Nevertheless, recent studies demonstrate that the direct use of this type of protein matrices may not be sufficiently adequate as they do not meet certain basic requirements to suitably favour cell support, such as insufficient enzymatic resistance, fast dissolution in water and low thermal and mechanical biostability. Other claims are based on the addition of synthetic polymers in the composition of the electrospun polymer (US Patent 0263417 A1, 2006), to in this way resolve the aforementioned problems. Nevertheless, many of these synthetic compositions lack sufficient thermal resistance to allow their sterilization and fixation by temperature. Unfortunately, the sterilization process is essential in any biomedical application since in this stage it totally eliminates the microbial load of a healthcare device to be able to use it aseptically in a surgical process. Thermal methods, both dry and moist heat, are usually the most suitable, although inconvenient, to sterilize any implant material or biomedical device.

On the other hand, the most typical final objective of electrospinning is to create a coating in the form of an interface in the implant and this is generally performed by means of the direct application on the implant of the electrospinning process. However, one of the most frequent problems of interfaces based on electrospun fibers is the difficulty in guaranteeing the bond between the implant and its coating, which may compromise the future integrity of the implant. Indeed, insufficient fixation between both could very considerably limit is application, given that they may not resist handling during surgery or later loads *in vivo* (Friess W. Collagen - biomaterial for drug delivery. European Journal of Pharmaceutics and Biopharmaceutics 45 (2): 113, 1998).

### DESCRIPTION OF THE INVENTION

The present invention provides a bioactive composition obtained through the technique of electrospinning which, due to its technical characteristics, may withstand thermal sterilization processes for their application in biomedical implants and tissue engineering.

A first aspect of the present invention relates to a bioactive composition obtained through an electrospinning process comprising at least one polymer which is selected from polyesters, polyketones, polysaccharides, thermally stable proteins or any of their mixtures.

In the present invention, bioactive is understood as the capacity a material has to induce, stimulate, provoke or modulate a biological action defined in the receptor tissue; hence, a bioactive material is that which enables a specific biological response in its interface of tissues, favouring the bonding of both.

The polyesters of the bioactive composition in a preferred embodiment are selected from a list comprising: polyglycolic acid (PGA), poly(glycolic-co-lactic) acid (PGLA), poly-L-lactic acid (PLLA), polyhydroxyalkanoates, polycaprolactones (PCL) and any of their combinations.

In another preferred embodiment, the polyketones of the bioactive composition are selected from a list comprising: polyether ether ketone (PEEK) and sulfonated polyether ether ketone (S-PEEK).

In another preferred embodiment the proteins of the bioactive composition are selected from a list comprising: zein, soy protein and any of their combinations.

In another preferred embodiment, the polysaccharides of the bioactive composition are selected from a list comprising: cellulose, chitosan and any of their combinations.

Additionally, the bioactive composition contains preferably at least one bioceramic material, more preferably being hydroxyapatite or other calcium and/or phosphorous based minerals, especially indicated for their application in bone tissues.

Also additionally, the bioactive composition preferably contains a drug, more preferably being an anti-inflammatory, an antibiotic, an analgesic or any of their combinations.

In a second aspect, the present invention relates to a process for obtainment of the aforementioned composition, comprising the following stages:
a. Homogenization of a precursor composition comprising a polymer which is selected from a polyester, a polyketone, a thermally stable protein, a polysaccharide or any of their combinations, and at least one solvent, the solvent more preferably being organic, an alcohol and even more preferably hexafluoro-2-propanol.
b. Static or dynamic electrospinning of the precursor composition obtained in stage (a) applying a difference in potential between the capillary containing the composition and the collector whereon it is deposited, which is oppositely charged. The high voltage applied is capable of generating a jet of the previously dissolved polymer, which in its course, is lengthened, hardened and dries (the solvent evaporates practically completely) to be collected in the collector in the form of fiber or another structure of ultrafine size.
c. Sterilization treatment of the composition obtained in stage (b).

In a preferred embodiment of the process, additionally, during stage (a), a bioceramic material, a drug or both is added, and more preferably between 0.001 and 90 % by weight with respect to the polymer(s).

Preferably the process furthermore comprises a post-treatment, which may be after stage (a), after stage (b) or after stage (c), and this post-treatment being a cross-linking reaction and/or washing: said cross-linking is performed both by chemical and physical methods, to facilitate the later implant-interface adhesion or improve the thermal resistance, and the washing is performed to eliminate any undesired components from the composition.

The homogenization of stage (a) is preferably performed by mechanical agitation, in accordance with the necessary conditions to dissolve the polymers used and the polymer(s) - solvent ration being of 0.01 to 95 % by weight.

The electrospinning of stage (b) is preferably performed in conditions wherein the distance between the capillary and the support is of 1 - 200 cm, a deposition rate between 0.001 - 100 ml/h and applying a voltage between 1 - 100 kV. More preferably, it is performed at a distance between 5 - 50 cm, a deposition rate between 0.01 - 10 ml/h and a voltage between 5 - 25 kV.

The electrospinning technique makes it possible to obtain coatings generally based on fibers of submicrometric size from a wide range of biocompatible and biodegradable polymers. Due to the fiber size, the electrospun coatings can mimic the real structure of live tissues and organs, which favours adhesion, growth, migration and cell differentiation. Furthermore, the materials with the greatest future as the base of implants are also polymeric, and can be both temporary and permanent.

This fiber electrospinning technique makes it possible to obtain fibers on a submicrometric or nanometric scale from a precursor composition. Electrospinning shares the characteristics of both electrospraying and the conventional solution of the dry spinning of fibers. The process is not invasive and does not require the use of coagulation chemistry or high temperatures to produce the fiber production. In electrospinning, the fibers produced are practically solvent-free. In some cases, traces thereof may remain, however they are usually totally eliminated during the thermal process of stage (c), so that no solvent is transferred to the end product.

The physicochemical characteristics of the composition of the present invention allow it to be sterilized by any known sterilization method. The possibility of sterilization, thanks to the high thermal resistance of the polymers used in the composition, is a great advantage, since in a preferred embodiment the sterilization treatment of stage (c) is performed by a thermal method, whether moist or dry, the moist method in a more preferred embodiment being performed in an autoclave at a temperature in a range from 60 to 200 °C during a time of at least 15 min. In an even more preferred embodiment, moist heat is applied in an autoclave at a temperature between 100 and 135 °C.

And in another more preferred embodiment the dry method of sterilization is performed in an oven at a temperature between 100 and 300 °C during a time of 1 minute to 4 hours. These thermal methods, both using dry and moist heat, are the most suitable and simplest to sterilize any material that is going to be implanted.

In some cases, it has been observed that the sterilization treatment at high temperatures may even increase the bioactivity of the coating. This could also produce a morphological change in the fibers that constitute the coating.

During the sterilization stage in (c), if this is performed using temperature, and if, furthermore, the implant whereon the electrospun fibers are coated is plastic or its surface has thermoplastic characteristics, it will be possible to fix the fibers to the implant additionally using heat. In this way it allows the development of composite materials, interface-plastic matrix, using electrospun coatings adhered to a polymeric matrix, establishing a physical or chemical bond between the two phases, through a thermal treatment. The bonding technique consists, in particular, of directly coating (in situ) or indirectly coating (on-, at- or off-situ) electrospun fibers on a thermostable element, without curing or pre-curing, and later subjecting the assembly to curing. As it is fixed to the implant, the coating is better able to resist mechanical and handling loads during surgery. Therefore, the use of a composite implant based on a polymeric matrix with electrospun fibers incorporated and fixed on its surface induces cellular growth, in general, and integrates the implant in the live tissue, in particular.

A third aspect of the present invention relates to the use of the aforementioned electrospun composition in biomedical applications. Where the biomedical applications are selected from a list comprising the manufacturing of prostheses and implants, the coating of implants, manufacturing of sutures, direct substitution of tissues, both of permanent and temporary replacement, in bones, skin, muscles and damaged organs, and as a controlled-release system of drugs.

"Implant", in the present invention, is understood to be a device, prosthesis or substance of a synthetic or natural material which is placed in the body with the intention of curing, correcting a health problem or with aesthetic purposes.

"Controlled-release system of drugs", in the present invention, is understood to be a manner of administering drugs locally, where the drug is incorporated in a necessary quantity in the implant coating, and is specifically released during a period of time in the place where the implant has been incorporated, acting effectively and precisely to favour a curing process and without the need to be applied systematically.

In a fourth aspect, the present invention relates to a material comprising a plastic matrix coated with the aforementioned bioactive electrospun composition.

The plastic matrix preferably comprises a thermostable polymer, an elastomeric polymer or a thermoplastic polymer.

The thermostable polymer is more preferably selected from the list comprising acrylic resins, epoxy resins, unsaturated polyesters, polyketones, phenolic resins, polyimides, polyurethanes, silicones, rubbers, natural gums or any of their combinations. This thermostable polymer even more preferably being cured, not cured or pre-cured.

In the case of thermoplastic polymers, the adhesion is achieved by thermal treatment followed by softening and diffusion in the interface and/or reaction between the implant and the coating. The thermoplastic polymers are more preferably selected from the list comprising biopolymers used in the biomedical area such as polyesters, polyols, polyurethanes, polyketones, biodegradable polyamides, polysaccharides and proteins. The biopolyesters are even more preferably: polylactic acid (PLA), polyglycolic acid (PGA), poly(hydroxybutanoates) (PHBs), poly(hydroxyalkoanates) (PHAs), polycaprolactones (PCL) or any of their combinations. And the polyols even more preferably: polysaccharides, polyvinyl alcohol (PVOH), its copolymers with ethylene (EVOH) or any of their mixtures.

In a fifth aspect, the present invention relates to a process for obtainment of the plastic matrix material coated with the bioactive electrospun composition, comprising the following stages:
a. coating a plastic matrix with the aforementioned composition.
b. performing a thermal adhesion treatment of the composition obtained in stage (a) over the matrix.

In a preferred embodiment of the process, the coating of stage (a) is carried out simultaneously to the electrospinning.

This process achieves an optimal adhesion of the coating on the implant, which is essential in clinical success.

Preferably, if the polymer is thermostable the thermal adhesion treatment is a curing treatment which is performed at a temperature range of 70 to 300 °C and during a time of 0.1 minutes to 5 hours.

The thermal curing treatment is not only going to give excellent adhesion between the electrospun fiber coating and the plastic implant, but it is also going to increase the cross-linking of the polymer.

Preferably, if the polymer is thermoplastic this thermal adhesion treatment is going to be performed at temperatures wherein the polymer is plasticized.

Preferably, stage (b) can also be a thermal sterilization process or, additionally, a sterilization stage can be carried out after this stage (b).

A sixth aspect of the present invention relates to the use of this plastic matrix material coated with the bioactive electrospun composition, as permanent or resorbable biocompatible implant.

The characteristics that the biocompatible implants must comply with in each case are:
- Permanent implants: In this case it is required that the polymer constituting the plastic matrix does not modify its physical properties during the life of the implant.
- Resorbable implants: Biodegradables polymers are used for this, both in the plastic matrix and in the interface, i.e. materials which, after fulfilling their function, biodegrade and are reduced to small molecules that are integrated in the typical biological cycles of the live organism. It is a temporary implant.

Throughout the description and the claims the word "comprises" and its variants are not intended to exclude other technical characteristics, additives, components or steps. For persons skilled in the art, other objects, advantages and characteristics of the invention will be inferred in part from the description and in part from the practice of the invention. The following figures and examples are provided by way of illustration, and are not intended to limit the present invention.

### DESCRIPTION OF THE FIGURES

**Fig. 1****.** Shows the PGLA fibers, before and after the thermal treatment, taken by an electronic scanning microscope.
**Fig. 2****.** Shows the cellular growth in accordance with time of both coatings, without treating and with heat, and of a film obtained through casting (formed by solvent evaporation) from the same polymeric solution used in the electrospinning process.
**Fig. 3****.** Shows the zein fibers, before and after the thermal treatment taken by an electronic scanning microscope.
**Fig. 4****.** Shows the results of the cell growth, showing a slight decrease in growth in the first few days after the thermal process, very possibly due to the larger size of the fibers.
**Fig. 5****.** Shows the acrylic resin before and after being coated with PGA fibers after fixation with temperature.
**Fig. 6****.** Shows in detail, by electronic scanning microscope, the morphology of PGA fibers that are coating an acrylic resin.
**Fig. 7****.** Shows the cell growth in accordance with time of an acrylic resin coated with electrospun PGA fibers. This growth was compared with that obtained in the same acrylic resin without coating and with the same coating of PGA fibers but on an inert support (aluminium foil). Furthermore, it compares the growth between the coating on the resin with that obtained on an inert support.
**Fig. 8** shows the PLA film before and after the electrospinning process plus the heating.
**Fig. 9****.** shows the cell growth in accordance with time of the PLA film coated with electrospun PGA fibers.

### EXAMPLES

Below, the invention will be illustrated with assays performed by the inventors, which reveal the specificity and efficacy of the bioactive composition obtained through the electrospinning process used individually or as interface between a plastic implant and the live tissue, in both cases inducing cell growth and the implant being integrated in the live tissue.

### Example 1

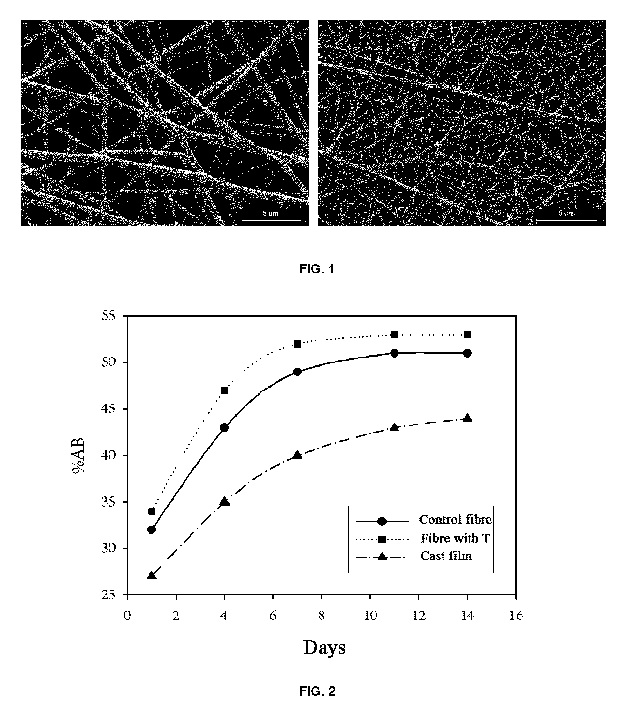

By way of example, the obtainment of an electrospun fiber coating of a polyester sterilized by moist heat is described. Initially, 5 g of poly(glycolic-co-lactic) acid (PGLA) are completely dissolved in 95 g of propanol hexafluoride. Then, the previously obtained polymeric solution is electrospun in the following conditions: 10 kV, 0.15 ml/h and 15 cm. Subsequently, the fibers are collected in a metal collector and are introduced in the autoclave at 121 °C during 20 min for their complete sterilization. The images of figure 1 show PGLA fibers, before and after the thermal treatment, taken by an electronic scanning microscope. These images show how the original fibrillar structure is maintained in the coating, in both cases below one micron, but the thermally treated fibers have a slightly smaller size and, furthermore, they have the appearance of porous morphology. Due to its topography, it is considered that the coating has biomimetic characteristics (it simulates the real structure of live tissues) and, therefore, high cellular bioactivity. Finally, the bioactivity was determined to verify the above, via an *in vitro* cellular viability assay. For this, osteoblasts (bone cells) of the *MG-63* cell line in *Eagle* medium enriched with nutrients are added to the coatings at a concentration of 3 x 10⁴. Adding Alamar Blue to the culture medium determines, by absorbance in the colour change from blue to red ( % AB), the number of metabolically active cells and, therefore, their growth. In this way, the graphic of figure 2 shows the cell growth in accordance with time of both coatings, untreated and with heat, and of a film obtained through casting (formed by solvent evaporation) from the same polymeric solution used in the electrospinning process. In said graphic it can be observed that cell growth even improves as a consequence of the thermal process applied. This may be due to the occurrence of a slight decrease in fiber size and also in the appearance of nanopores that could favour cell anchorage. In this way, it presents an improvement in the bioactivity established with respect to the same material in the form of a flat surface film.

### Example 2

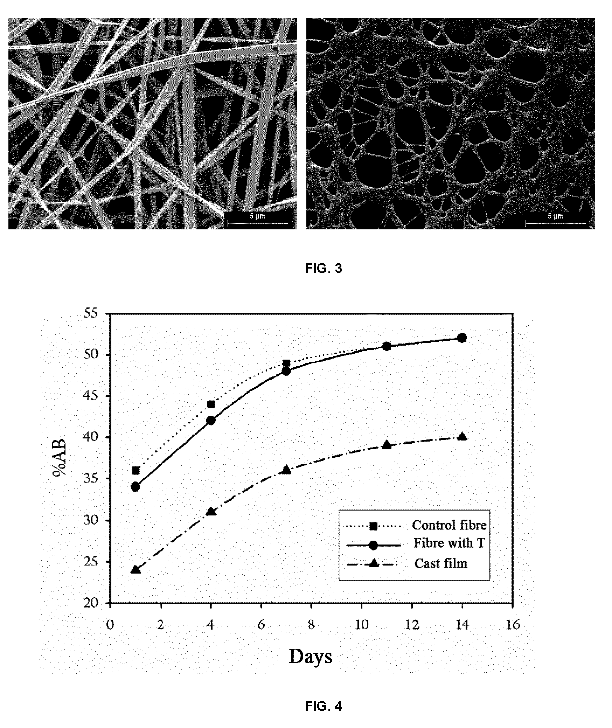

The following example relates to the obtainment of a similar coating but, in this case, obtained for a protein and sterilized by heat dry. For this, 33 g of zein are completely dissolved in a mixture of 64 g ethanol and 11 g water. 1.5 g of citric acid is added to this polymeric solution in the presence of 0.5 g of sodium hypophosphite monohydrate as cross-linking agents. Then, the previously obtained polymeric solution is electrospun in the following conditions: 12 kV, 0.10 ml/h and 13 cm. Subsequently, the fibers are collected in a metal collector and are introduced in an oven at 180 °C during 30 min for their complete sterilization. The images of figure 3 show the zein fibers, before and after the thermal treatment taken by an electronic scanning microscope. As in the previous example, it shows how the coatings continue to maintain their submicrometric fibrillar structure. Nevertheless, a cross-linking process is observed in the thermally treated structure whereby the fibers undergo a certain widening and form a much more compact framework. As a consequence of this new structure, the zein fibers have an optimized water resistance and better physical properties. Bioactivity assays with the osteoblasts, according to the process described in example 1, are performed on both samples and a zein film obtained through casting from the same polymeric solution. The graphic of figure 4 show the cell growth results, showing that there is a slight decrease in growth in the first few days after the thermal process very possibly due to the greater size of the fibers. Nevertheless, the improvement in bioactivity with respect to the film continues to be very considerable.

### Example 3

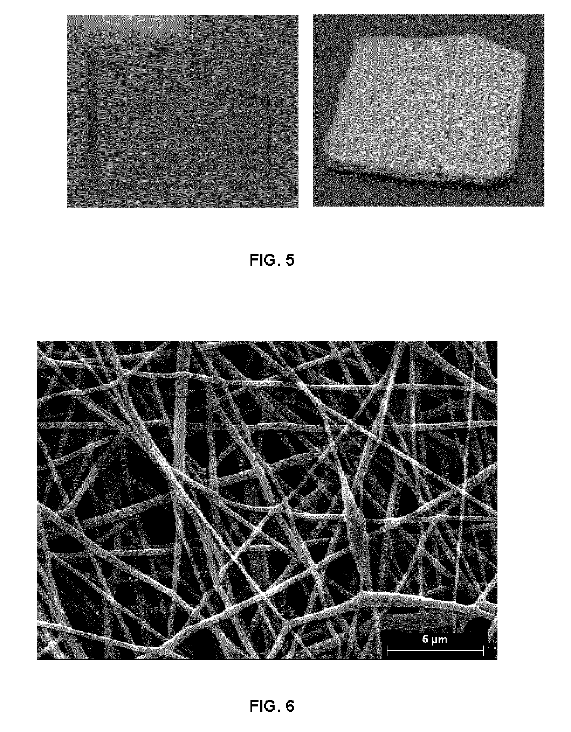

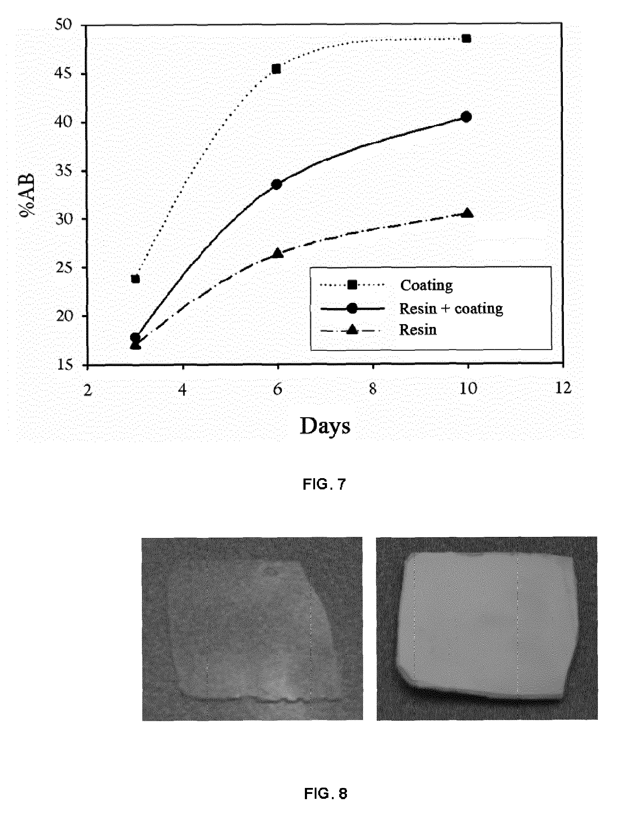

The obtainment of an electrospun fiber coating of a polyester adhered to an acrylic resin is described. Initially, 5 g of polyglycolic acid (PGA) are completely dissolved in 95 g of propanol hexafluoride. Then, the previously obtained polymeric solution is electrospun on an acrylic resin pre-cured at 100 °C during 30 min. The electrospinning process is performed in the following conditions: 9 kV, 0.20 ml/h and 12 cm. Subsequently, the resin coated superficially with the electrospun fibers is introduced in an oven at 150 °C during 2 h. The image of figure 5 shows the acrylic resin before and after being coated with the PGA fibers after fixation with temperature. Figure 6 shows in detail, by electronic scanning microscope, the morphology of the surface of the acrylic resin with the PGA fibers which coat it. As can be observed visually, the resulting composite material has a surface based on electrospun fibers, which simulate the real morphology of live tissues. The bioactivity of the surface, combined with the rigidity of the resin, gives this material ideal properties for its use as an implant element. Finally, to verify the improvement in bioactivity, it was possible to determine the cellular viability using an *in vitro* cellular viability assay. For this, osteoblasts (bone cells) of the *MG-63* cell line in *Eagle* medium enriched with nutrients are added to the coatings at a concentration of 3 x 10⁴. By adding Alamar Blue to the culture medium it determines, in accordance with example 1, the number of metabolically active cells and, therefore, their growth. In this way, the graphic of figure 7 shows cell growth in accordance with time of the acrylic resin coated with electrospun PGA fibers. This growth is compared with that obtained in the same acrylic resin without coating and with that of the same coating of PGA fibers but on an inert support (aluminium foil). In said graphic it can be observed that cell growth greatly improves on coating the resin with the electrospun fibers. Indeed, we compare the growth between the coating on the resin and that obtained on an inert support a decrease can be observed, very possibly due to the low biocompatibility of the resin which could be associated with slight cytotoxicity. In this way, it presents a process capable of generating a permanent biocompatible implant based on an interface-plastic matrix with improved bioactivity.

### Example 4

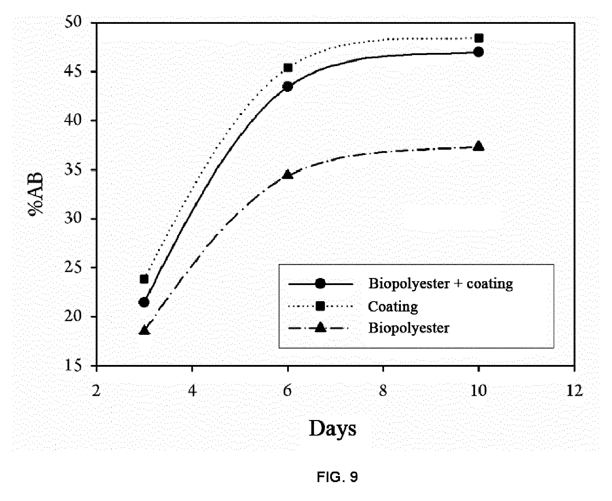

The obtainment of an electrospun fiber coating of a polyester adhered to a thermoplastic is described. The polymeric solution obtained in example 3 is electrospun in the same conditions on a polylactic acid (PLA) film previously obtained through casting (formation by evaporation of the solvent). Subsequently, the PLA film coated superficially with the electrospun fibers is introduced in an oven at 110 °C during 30 minutes. The image of figure 8 shows the PLA film before and after the electrospinning process plus the heating. In the same way, to verify the improvement in bioactivity the cellular capacity was determined via an *in vitro* cellular viability assay with osteoblasts using the Alamar Blue method described in example 1. Thus the graphic of figure 9 shows cell growth in accordance with time of the PLA film coated with electrospun PGA fibers. This growth is compared with that obtained in the same acrylic resin without coating and with that of the same coating of PGA fibers but on an inert support (aluminium foil). In said graphic it can be observed that cell growth greatly improves after coating the PLA film with the electrospun PGA fibers. This is due to the surface of the film being completely smooth so that bioactivity is not increased unlike the electrospun fibers that simulate the real morphology of live tissues. However, given that PLA is completely biocompatible and has no associated cytotoxicity, the growth between the coating on PLA with that obtained on an inert support is practically the same. In this way, a process is presented capable of generating a temporary biocompatible implant with improved bioactivity.

## Claims

1. Bioactive composition obtained through an electrospinning process comprising at least one polymer which is selected from
● polyesters,
● polyketones,
● thermally stable proteins,
● polysaccharides
● or any of their mixtures.

2. Bioactive composition according to claim 1, where the polyesters are selected from a list comprising: polyglycolic acid (PGA), poly(glycolic-co-lactic) acid (PGLA) and poly-L-lactic acid (PLLA), polyhydroxyalkanoates and polycaprolactones (PCL).

3. Bioactive composition according to claim 1, where the polyketones are selected from a list comprising: polyether ether ketone (PEEK) and sulfonated polyether ether ketone (S-PEEK).

4. Bioactive composition according to claim 1, where the proteins are selected from a list comprising: zein, soy protein and any of their combinations.

5. Bioactive composition according to claim 1, where the polysaccharides are selected from a list comprising: cellulose, chitosan or any of their combinations.

6. Bioactive composition according to any of claims 1 to 5, which further comprises a bioceramic material.

7. Bioactive composition according to any of claims 1 to 6, which further comprises a drug.

8. Bioactive composition according to claim 7, where the drug is selected from the list comprising: anti-inflammatory, antibiotic, analgesic or any of their combinations.

9. Process for obtainment of the composition according to any of claims 1 to 8, comprising the following stages:
a. Homogenization of a precursor composition comprising a polymer which is selected from a polyester, a polyketone, a thermally stable protein, a polysaccharide or any of their mixtures, and at least one solvent;
b. Static or dynamic electrospinning of the composition obtained in stage (a) applying a difference in potential between the capillary containing the composition and the collector whereon it is deposited,
c. Sterilization of the composition obtained in stage (b).

10. Process according to claim 9, where at least one bioceramic material, a drug or both is added during stage (a).

11. Process according to any of claims 9 or 10, where at least one post-treatment is performed which may be after stage (a), after stage (b) or after stage (c).

12. Process according to claim 11, where the post-treatment comprises a process of cross-linking and/or washing.

13. Process according to any of claims 9 to 12, where the homogenization of stage (a) is performed by mechanical agitation.

14. Process according to any of claims 9 to 13, where the electrospinning of stage (b) is performed at a distance between the capillary and the support of between 1 and 200 cm.

15. Process according to claim 14, where the electrospinning of stage (b) is performed at a distance between the capillary and the support of between 5 and 50 cm.

16. Process according to any of claims 9 to 15, where the electrospinning of stage (b) is performed at a deposition rate between 0.001 and 100 ml/h.

17. Process according to claim 16 where the electrospinning of stage (b) is performed at a deposition rate between 0.01 and 10 ml/h.

18. Process according to any of claims 9 to 17, where the electrospinning of stage (b) is performed applying a voltage between 1 and 100 kV.

19. Process according to claim 18, where the electrospinning of stage (b) is performed applying a voltage between 5 and 25 kV.

20. Process according to any of claims 9 to 19, where the sterilization treatment of stage (c) is performed by application of moist or dry heat.

21. Process according to claim 20, where the moist heat is applied in an autoclave at a temperature between 60 and 200 °C during a time of at least 15 min.

22. Process according to claim 21, where the moist heat is applied in an autoclave at a temperature between 100 and 135 °C.

23. Process according to claim 20, where the dry method is performed in an oven at a temperature between 100 and 300 °C during a time of 1 minute to 4 h.

24. Use of the electrospun composition according to any of claims 1 to 8 in biomedical applications.

25. Use of the electrospun composition according to claim 24, where the biomedical application is selected from a list comprising the manufacturing of prostheses and implants, the coating of implants, manufacturing of sutures, direct substitution of tissues and controlled-release systems of drugs.

26. Material comprising a plastic matrix coated by a composition according to any of claims 1 to 8.

27. Material according to claim 26, where the plastic matrix comprises a thermostable polymer, an elastomeric polymer or a thermoplastic polymer.

28. Material according to claim 27, where the plastic matrix is a thermostable or elastomeric polymer which is selected from the list comprising acrylic resins, epoxy resins, polyurethanes, unsaturated polyesters, polyketones, polyphenolic resins, polyimides, polyurethanes, silicones, rubbers, natural gums or any of their combinations.

29. Material according to claim 28, where the thermostable or elastomeric polymer is cured, not cured or pre-cured.

30. Material according to claim 27, where the plastic matrix is a thermoplastic polymer which is selected from the list comprising polyesters, polyols, polyketones, polyamides biodegradables, polysaccharides, proteins and polyolefins.

31. Material according to claim 30, where the thermoplastic polymer is a polyester which are selected from the list comprising polylactic acid (PLA), polyglycolic acid (PGA), poly(hydroxyalkoanates) (PHAs), polycaprolactones (PCL) poly(hydroxyalkoanates) (PHAs) or any of their combinations.

32. Material according to claim 30, where the thermoplastic polymer is a polyol which is selected from the list comprising polysaccharides, polyvinyl alcohol (PVOH), its copolymers with ethylene (EVOH) or any of their combinations.

33. Process for obtainment of the material according to any of claims 26 to 32, comprising:
a. coating a plastic matrix with the composition according to any of claims 1 to 8,
b. performing a thermal adhesion treatment of the composition over the matrix.

34. Process according to claim 33, where the coating of stage (a) is carried out simultaneously with an electrospinning process.

35. Process according to any of claims 33 or 34, where the thermal adhesion treatment, when the plastic matrix is a thermostable polymer, is a process of curing or post-curing.

36. Process according to any of claims 33 or 34, where the thermal adhesion treatment, when the plastic matrix is a thermoplastic polymer, is performed at temperatures wherein the polymer is plasticized.

37. Process according to any of claims 33 to 36, which further comprises a sterilization stage after stage (b).

38. Use of the material according to any of claims 26 to 32, as permanent or resorbable biocompatible implant.
